# EUROPÄISCHE PATENTANMELDUNG

(11) **EP 2 163 620 A1**
(43) Veröffentlichungstag der Anmeldung: **17.03.2010**
(21) Anmeldenummer: 08163623.5
(22) Anmeldetag: 03.09.2008
(51) Int. Cl.: C12N 15/09

(54) **Verfahren zum Isolieren und Reinigen von Nukleinsäuren**

(71) Anmelder: QIAGEN GmbH, 40724 Hilden (DE)
(72) Erfinder: Die Erfindernennung liegt noch nicht vor
(74) Vertreter: Vossius & Partner

(57) **Zusammenfassung**

Die gegenwärtige Erfindung betrifft ein Verfahren zum Isolieren und Reinigen von Nukleinsäuren durch Elution der Nukleinsäuren von Nukleinsäurehaltigen Proben, sowie biologischen Materialien. Die gegenwärtige Erfindung betrifft weiterhin einen Kit zur Durchführung des erfindungsgemäßen Verfahrens.

## Beschreibung

Die vorliegende Erfindung betrifft ein Verfahren zum Isolieren und Reinigen von Nukleinsäuren durch Elution der Nukleinsäuren von Nukleinsäurehaltigen Proben, sowie biologischen Materialien. Die vorliegende Erfindung betrifft weiterhin einen Kit zur Durchführung des erfindungsgemäßen Verfahrens.

Ein effizientes Verfahren zum Isolieren und Reinigen von Nukleinsäuren, welches im Stand der Technik bereits bekannt ist, basiert auf der Adsorption von Nukleinsäuren auf Glas- oder Silikapartikeln in Anwesenheit chaotroper Salze, gefolgt von der Wiedergewinnung der adsorbierten Nukleinsäuren (Vogelstein, B. und Gillespie, D. (1979); "Preparative and analytical purification of DNA from agarose", Proc. Natl. Acad. Sci. USA 76: 615-619). Gemäß diesem Verfahren wird unter Verwendung hoher Konzentrationen chaotroper Salze, wie beispielsweise Natriumiodid, Natriumperchlorat oder Guanidiniumthiocyanat, DNA isoliert und über Agarose gereinigt. Die RNA oder DNA kann auch aus verschiedenen Mischungen isoliert oder gereinigt werden (Boom, R. (1990); "Rapid and simple method for purification of nucleic acids", J. Clin. Microbiol. 28: 495-503).

Nach erfolgter Reinigung werden Nukleinsäuren häufig in der Polymerasekettenreaktion (PCR) verwendet. Die PCR amplifiziert Polynukleinsäuren in einer Sequenz spezifischen Weise und wird deshalb breit in der Gen- oder DNA-Diagnose angewendet. Die Anwendung der PCR-Technik in klinischen Routineverfahren bringt mehrere Probleme mit sich. Es ist bekannt, dass inhibitorische Substanzen, die nicht aus dem gereinigten Nukleinsäure-Präparat entfernt worden sind, die PCR inhibieren können. Solche inhibitorischen Substanzen sind beispielsweise Hämoglobin und Tenside, die im Nukleinsäureextraktionsprozess verwendet wurden. Aus diesem Hintergrund wird ersichtlich, dass die Verfahren zum Extrahieren und Reinigen von Nukleinsäuren sehr wichtig und relevant sind (Oshima et al., JJCL A, 22(2) 145-150(1997)).

Verfahren zur Extraktion und Reinigung von Nukleinsäuren werden sehr häufig automatisiert.

Der Stand der Technik kennt bereits automatisierte Nukleinsäureextraktionsverfahren, solche sind beschrieben in JP-A-107854/1999 und in JP-A-266864/1999. In den meisten Verfahren zur Isolation und Reinigung von Nukleinsäuren wird eine Lösung, die in hoher Konzentration Salze enthält und in hoher Konzentration Alkohol enthält, und in welcher sich die Nukleinsäuren befinden, in Kontakt mit einer Adsorptionsoberfläche gebracht. Die Adsorptionsoberfläche kann hierbei eine Säule sein. Dann werden die Nukleinsäuren an diese Oberfläche adsorbiert und später mit Hilfe von Lösungen eluiert, die weniger hoch konzentrierte Salzlösungen enthalten.

Das Problem der meisten Verfahren zur Isolation und Reinigung von Nukleinsäuren ist, dass die Ausbeute der Nukleinsäuren relativ gering ist. Ein weiteres Problem ist, dass ethanolhaltige Lösungen gemäß den Regularien der IATA (International Air Transport Association) als gefährliche Materialien eingestuft werden (HAZMAT; Hazardous Materials). Gemäß den IATA Regularien werden alle Produkte, Materialien und Güter in neun Hauptklassen klassifiziert. Durch die Einstufung als gefährliche Güter werden beim Transport durch Flugzeuge zusätzliche Gebühren und Steuern fällig. Aufgabe der gegenwärtigen Erfindung war es daher, es daher, Ethanol (oder Isopropanol) im Verfahren zur Reinigung und Extraktion von Nukleinsäuren weitgehend zu ersetzen, um die Isolation und Reinigung von Nukleinsäuren zu erleichtern, ein Ethanolfreies Verfahren bereitzustellen und den Luftfrachttransport zu erleichtern.

Aus dem Stand der Technik sind Ersatzstoffe für Alkohol in Verfahren zur Reinigung von Nukleinsäuren bekannt, die jedoch die oben besprochen Probleme nur teilweise lösen (US 2004/0167324). Die Mehrheit der hierin beschriebenen Substanzen fallen entweder unter die HAZMAT IATA Regularien oder weisen einen stechenden Geruch auf, so dass ihre Verwendung nur im Abzug erlaubt ist.

Um die oben genannten Probleme besser zu lösen, bestand der Bedarf nach weiteren Alkohol-Ersatzstoffen in Verfahren zur Isolation und Reinigung von Nukleinsäuren.

Gegenstand der gegenwärtigen Erfindung ist ein Verfahren zur Extraktion von Nukleinsäuren aus einer Lösung umfassend die Schritte:
(a) Hinzufügen eines Bindungsmediators zu der Nukleinsäure enthaltenden Lösung,
(b) in Kontakt bringen der Lösung, welche den Bindungsmediator und die Nukleinsäuren enthält, mit einer Oberfläche unter chaotropen und/oder Hoch-Salz-Bedingungen
(c) Bindung bzw. Adsorption der Nukleinsäuren an eine Oberfläche,
(d) Waschen der Oberfläche durch einen Waschpuffer,
(e) Wiedergewinnung der Nukleinsäuren, welche an die Oberfläche adsorbiert sind, durch Elution,
   **dadurch gekennzeichnet, dass** der Bindungsmediator ausgewählt ist aus der Gruppe umfassend Diethylenglycolmonoethylether, Diethylenglycolmonoethyletheracetat, Furfurylalkohol, Poly(1-vinylpyrrolidon-co-2-dimethylaminoethylmethacrylat), Poly(2-ethyl-2-oxazolin), Poly(4-ammonium-stryolsulfonsäure), Tetraethylenglycoldimethylether, Tetraethylenglycol, Tetrahydrofurfuryl-polyethylenglycol 200 und Triethylenglycolmonoethylether.

Der Fachmann kann auch durch Gemische der genannten Bindungsmediatoren erfolgreich Ethanol im Bindungsprozess bei der Nukleinsäurepräparation ersetzen. Da ethanolhaltige Lösungen bis 24% (vol/vol) nicht als HAZMAT klassifiziert werden, können auch Gemische der Bindungsmediatoren mit Ethanol eingesetzt werden

Die Bindungsmediatoren liegen bevorzugt in folgenden Konzentrationen vor:
Diethylenglycolmonoethylether (DGME) [CAS 111-90-0] - Konzentrationsbereich 70-99%, bevorzugte Konzentration 99,0%; in Kombination mit Ethanol: 60-80% DGME und 16-24% Ethanol
Diethylenglycolmonoethyletheracetat (DGMEA) [CAS 112-15-2] - Konzentrationsbereich 70-99%, bevorzugte Konzentration 99,0%; in Kombination mit Ethanol: 60-80% DGMEA und 16-24% Ethanol
Furfurylalkohol [CAS 98-00-0] - Konzentrationsbereich 20-30%, bevorzugte Konzentration 30%
Poly(1-vinylpyrrolidon-co-2-dimethylaminoethylmethacrylat) [CAS 30581-59-0) - Konzentrationsbereich 3-5%, bevorzugte Konzentration 5%
Poly(2-ethyl-2-oxazolin) [CAS 25805-17-8] - Konzentrationsbereich 9-15% (w/v), bevorzugte Konzentration 12%; in Kombination mit Ethanol: 22,5% (w/v) und 16-24% (v/v) Ethanol
Poly(4-ammonium-styrolsulfonsäure) Konzentrationsbereich 8-22% (w/v), bevorzugte Konzentration 12%; in Kombination mit Ethanol: 8-22 (w/v) und 24% (v/v) Ethanol
Tetraethylenglycoldimethylether [CAS 143-24-8] - Konzentrationsbereich 70-98%, bevorzugte Konzentration 98%; in Kombination mit Ethanol: 73,5% und 24% Ethanol
Tetraglycol [CAS 9004-76-6] - bevorzugte Konzentration mit Ethanol: 75% und 16-24% Ethanol
Tetrahydrofurfurylpolyethylenglycol 200 [CAS 31692-85-0] - Konzentrationsbereich 70-100%, bevorzugte Konzentration 100%
Triethylenglycolmonoethylether [CAS 112-50-5] - Konzentrationsbereich 70-90%, bevorzugte Konzentration 90%

Die Bindungsmediatoren der vorliegenden Erfindung werden nach IATA überwiegend als ungefährlich eingestuft. Zudem wurden gute Ausbeuten mit den erfindungsgemäßen Bindungsmediatoren erreicht (siehe Beispiel 9).

Die nukleinsäurehaltige Lösung kann durch einen Lysevorgang aus einem Nukleinsäure enthaltenden biologischen Probenmaterial gewonnen sein. Dieses Probenmaterial kann beispielsweise Blut, Gewebe, Abstrichpräparate, Bakterien, Zellsuspensionen, Urin und adherierende Zellen sein. Das Nukleinsäure enthaltende Material kann menschliches, tierisches oder pflanzliches Material sein.

Die nukleinsäurehaltige Lösung kann aus einer biochemischen Nukleinsäuremodifizierungsreaktion gewonnen sein oder aus der Polymerase-Kettenreaktionen abstammen.

Die Nukleinsäure kann beispielsweise genomische DNA, Gesamt-DNA, oder kurze doppelsträngige DNA Fragmente sein.

In einer bevorzugten Ausführungsform ist die Nukleinsäure genomische DNA.

In einer anderen bevorzugten Ausführungsform ist die Nukleinsäure Gesamt-RNA.

In einer weiteren bevorzugten Ausführungsform sind die Nukleinsäuren kurze doppelsträngige DNA Fragmente.

In einer bevorzugten Ausführungsform ist die Nukleinsäure-enthaltende Lösung durch einen Lysevorgang aus einem Nukleinsäure enthaltenden Material gewonnen worden.

In einer anderen bevorzugten Ausführungsform ist die Nukleinsäure-enthaltende Lösung aus einer biochemischen Nukleinsäuremodifizierungsreaktion gewonnen worden.

Chaotrope Bedingungen werden durch die Zugabe chaotroper Substanzen erreicht. Als chaotrop werden chemische Substanzen bezeichnet, die geordnete Wasserstoff-Brückenbindungen in wässrigen Lösungen verhindern. Sie vermindern damit den hydrophoben Effekt und wirken denaturierend auf Proteine, da die treibende Kraft der Proteinfaltung die Zusammenlagerung der hydrophoben Aminosäuren im Wasser ist. Beispiele für chaotrope Substanzen sind Bariumsalze, Guanidiniumhydrochlorid, Thiocyanate wie Guanidiniumthiocyanat, Perchlorate oder auch Natriumchlorid. Chaotrope Salze können gemäß ihres Löslichkeitsproduktes in Konzentrationsbereichen zwischen 1 M und 8 M eingesetzt werden.

Hoch-Salz Bedingungen bedeutet hochkonzentrierte Salzlösungen, wobei die Konzentration der Salze in der Lösung mindestens 1 M beträgt, bevorzugt 1-4M beträgt.
Es können jedoch auch alternative Maßnahmen getroffen werden, um zu chaotropen oder Hoch-Salz-Bedingungen gelangen; die den gleichen Effekt erzielen, nämlich die Bindung der zu reinigenden Nukleinsäuren an die Oberfläche.

Die Oberfläche, an welche die Nukleinsäuren adsorbiert werden, basiert auf Materialien, die aus der folgenden Gruppe ausgewählt sind: Silika-Materialien, carboxylierte Oberflächen, Zeolithe und Titandioxid.

Bevorzugt gemäß der vorliegenden Erfindung ist ein erfindungsgemäßes Verfahren, **dadurch gekennzeichnet, dass** chaotrope Bedingungen durch den Zusatz chaotroper Salzen wie Kaliumiodid, Guanidiniumhydrochlorid, Guanidiniumthiocyanat oder Natriumchlorid zu der Nukleinsäure enthaltenden Lösung erreicht werden.

Es ist bevorzugt, dass der Nukleinsäure enthaltenden Lösung Tenside zugesetzt werden. Diese Tenside werden bevorzugt im Konzentrationsbereich von 0,1 Vol% bis 10 Vol% eingesetzt. Des Weiteren können Schaumbildungs-verhindernde Agentien (Anti-Foams) zugesetzt werden, bevorzugt in einem Bereich von 0,01 Gew% bis 1 Gew%.

Waschpuffer und Elutionspuffer, die für die erfindungsgemäßen Verfahren eingesetzt werden können, sind dem Fachmann bekannt.
Waschpuffer enthalten organische Lösungsmittel wie z.B. Alkohol. Waschpuffer entfernen die anderen Komponenten aus den Nukleinsäure enthaltenden Lösungen (andere als die Nukleinsäuren selbst).
Elutionspuffer sind in der Regel gepufferte Niedrigsalzlösungen mit neutralem bis leicht alkalischem pH Wert (zB: Puffer TE der Firma QIAGEN GmbH, Hilden). Der Fachmann benutzt zum Teil auch destilliertes Wasser.

Gegenstand der vorliegenden Erfindung ist ein Reagenzien-Kit zur Extraktion von Nukleinsäuren aus einer Lösung umfassend
- eine Lösung 1 umfassend den Bindungsmediatior ausgewählt aus der Gruppe umfassend Diethylenglycolmonoethylether, Diethylenglycolmonoethyletheracetat, Furfurylalkohol, Poly(1-vinylpyrrolidon-co-2-dimethylaminoethylmethacrylat), Poly(2-ethyl-2-oxazolin), Poly(4-ammonium-styrolsulfonsäure), Tetraethylenglycol-dimethylether, Tetraethylenglycol, Tetrahydrofurfuryl-polyethylenglycol 200 und Triethylenglycolmonoethylether und gegebenenfalls
- eine Lösung 2 umfassend Waschpuffer, und gegebenenfalls
- eine Lösung 3 umfassend ein Elutionsmittel.

Ein Kit der Firma QIAGEN für die Reinigung von Nukleinsäuren aus biochemischen Nukleinsäuremodifikationsreaktion würde beispielsweise neben den erwähnten Bindungsmediatoren folgende Bestandteile aufweisen:
Adsorptive Medien: QIAGEN (QIAamp^{®}; RNeasy^{®}; QIAquick^{®}) Spin Columns oder magnetische Silica-Partikel ("MagAttract® Suspension G")
Bindungspuffer: Bestehend aus chaotropern Salz und Bindungsmediatoren
Waschpuffer: "Puffer PE" (Pufferbeschreibung siehe Tabelle I)
Elutionspuffer: "Puffer AE", "Puffer EB"; "Puffer TE"; "RNase-freies Wasser"

Ein Kit der Firma QIAGEN für die Reinigung von Nukleinsäuren aus biologischen Probematerialien würde beispielsweise neben den erwähnten Bindungsmediatoren folgende Bestandteile aufweisen:
Adsorptive Medien: QIAGEN (QIAamp^{®}; RNeasy^{®}; DNeasy^{®}; QIAprep^{®}) Spin Columns oder magnetische Silica-Partikel ("MagAttract Suspension G")
Lyse Puffer: "Puffer AL"; "Puffer RLT"; "Puffer ATL"; "Puffer ML"; "Puffer AP1"; oder andere bereits kommerziell erhältliche Puffer
Protease: "QIAGEN Protease"; Proteinase K; Lysozym und andere proteolytische Enzyme
Waschpuffer: "Puffer AW1"; "Puffer AW2"; "Puffer RW1"; "Puffer RPE"; oder andere bereits kommerziell erhältliche Puffer
Elutionspuffer: "Puffer AE", "Puffer EB "; "Puffer TE"; "RNase-fieies Wasser"

Entsprechende Lysepuffer sind dem Fachmann bekannt und enthalten in der Regel Detergenzien, Chelatoren für divalente Kationen, pH Puffersubstanzen und chaotrope Salze.

Der erfindungsgemäße Reagenzien-Kit zur Extraktion von Nukleinsäuren kann in einer bevorzugten Ausführungsform Waschpuffer und Elutionspuffer, wie in WO 99/22021, EP 1 121 460 und US 7,074,916 beschreiben, enthalten. Die darin beschriebenen Waschpuffer und Elutionspuffer sind Bestandteil dieser Offenbarung.

Der erfindungsgemäße Reagenzien-Kit zur Extraktion von Nukleinsäuren kann in einer bevorzugten Ausführungsform als Elutionsmittel zum Beispiel: "Puffer TE" oder auch destilliertes Wasser enthalten.

In einer bevorzugten Ausführungsform enthält der erfindungsgemäße Reagenzienkit zur Extraktion von Nukleinsäuren aus einer Lösung ein chaotropes Salz in einer Pufferlösung. Der Kit enthält also beispielsweise einen chaotropen Puffer, einen Lysepuffer und einen Bindungsmediator.

Bevorzugtermaßen ist das chaotrope Salz ausgewählt aus einer Gruppe umfassend Natriumiodid, Guanidiniumhydrochlorid, Guanidiniumthiocyanat; Natriumperchlorat und Natriumchlorid.

Gegenstand der vorliegenden Erfindung ist weiterhin die Verwendung des erfindungsgemäßen Reagenzien-Kits für die Aufreinigung von Nukleinsäuren aus biologischen Materialien wie Blut, Geweben, Abstrichpräparaten, Bakterien, Zellsuspensionen und adherierenden Zellen.

Gegenstand der vorliegenden Erfindung ist weiterhin die Verwendung des erfindungsgemäßen Reagenzien-Kits für die Aufreinigung von Nukleinsäuren aus biochemischen Reaktionen, PCR-Reaktionen und *in vitro*-Nukleinsäuremodifikationensreaktionen.

Bei den in der vorliegenden Anmeldung beschriebenen Produkten, Puffern und Protokollen (Verfahrensanleitungen) handelt es sich um publizierte Dokumente und kommerziell erhältliche Produkte der Firma QIAGEN GmbH, Hilden, Deutschland, wenn nicht anders gekennzeichnet.

### Beschreibung der Figuren

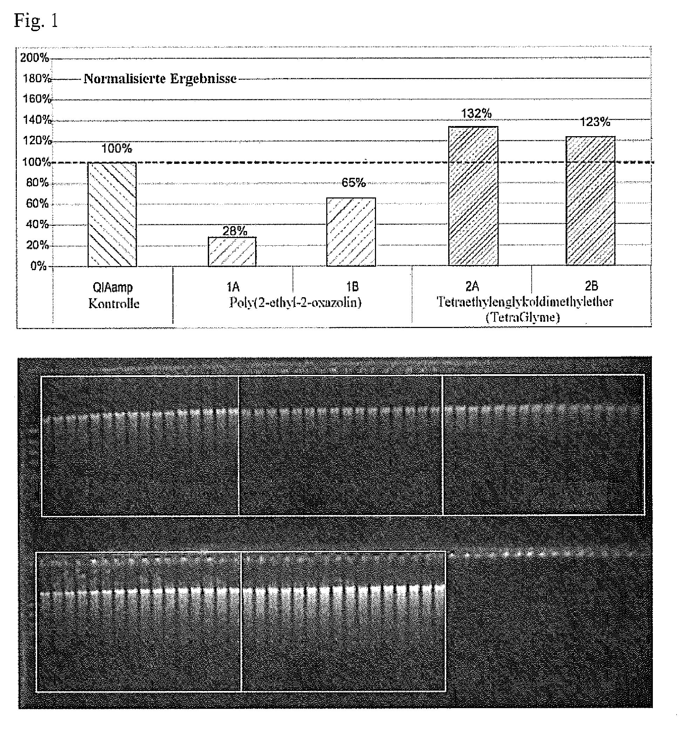

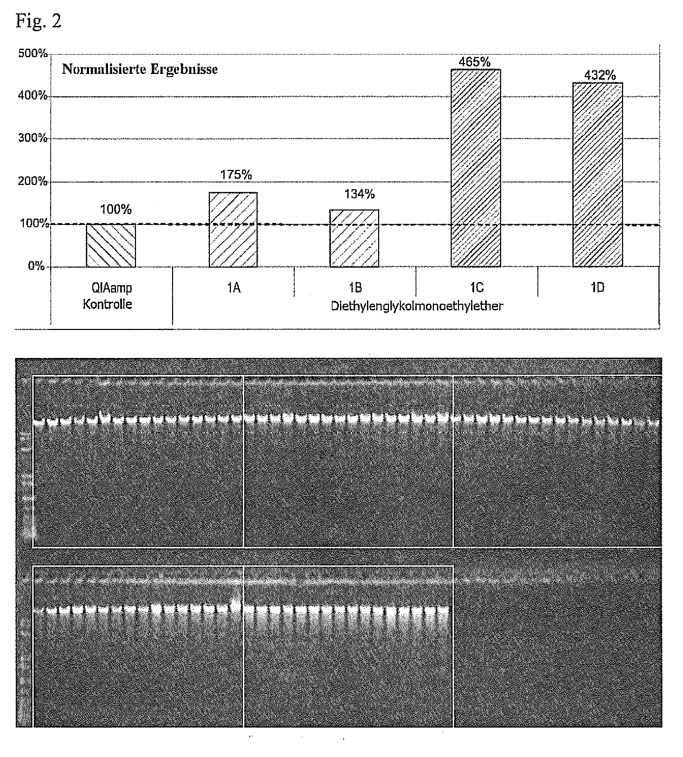

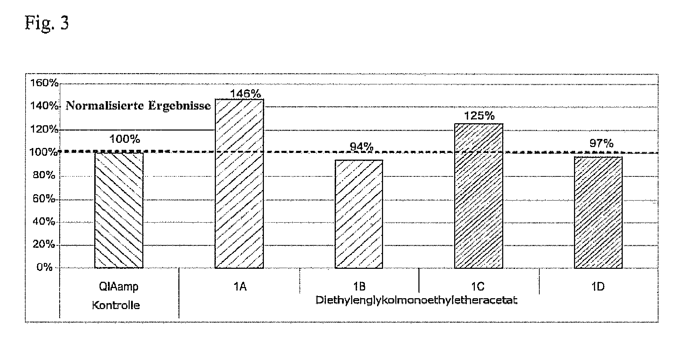

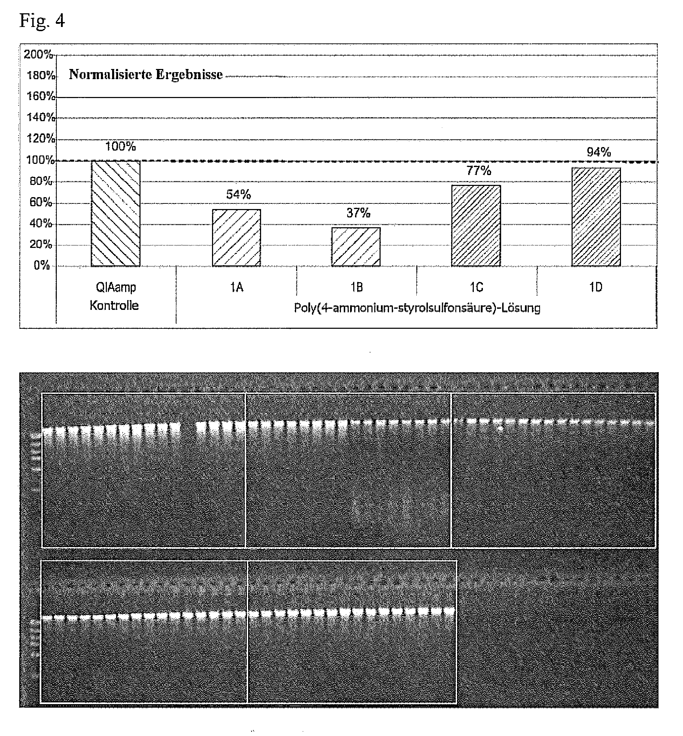

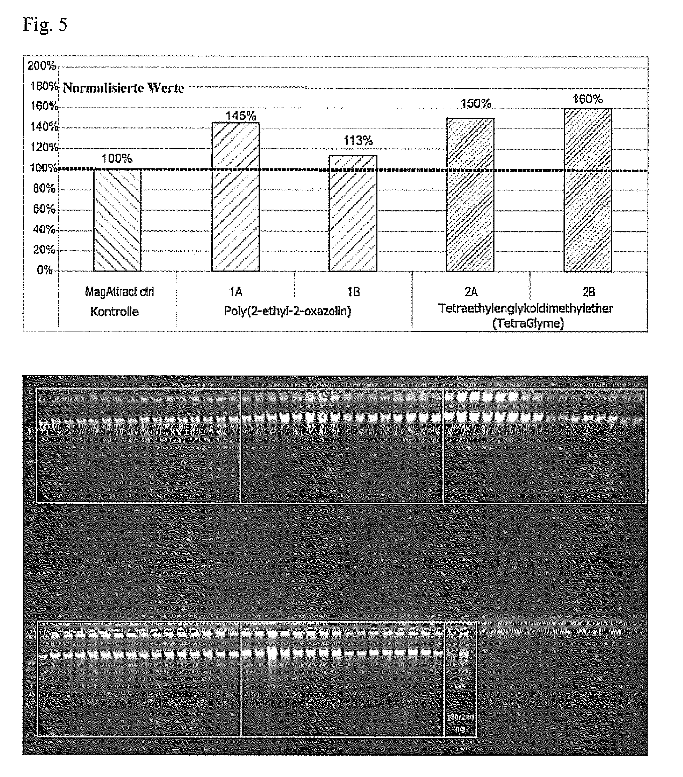

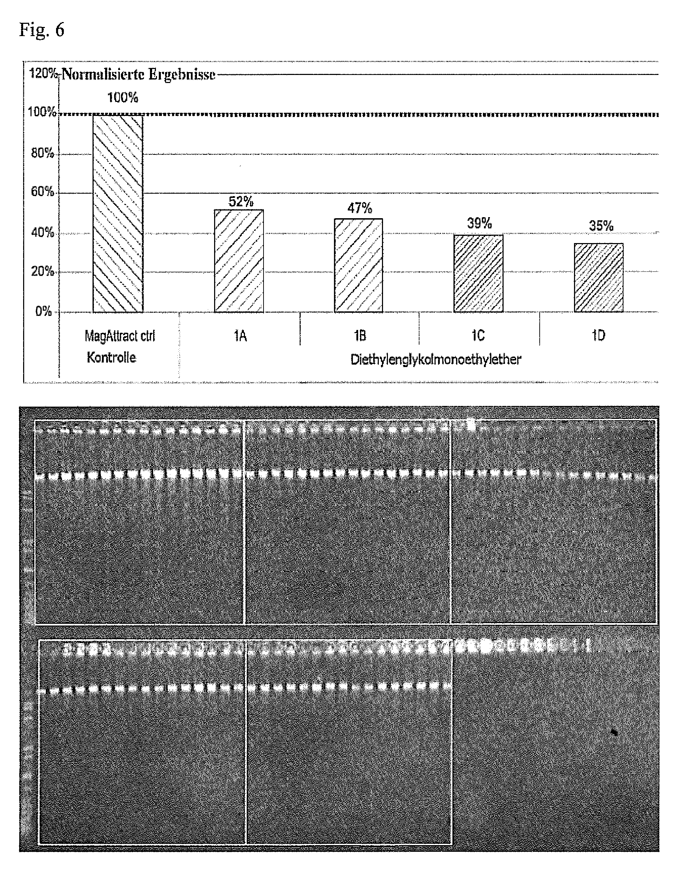

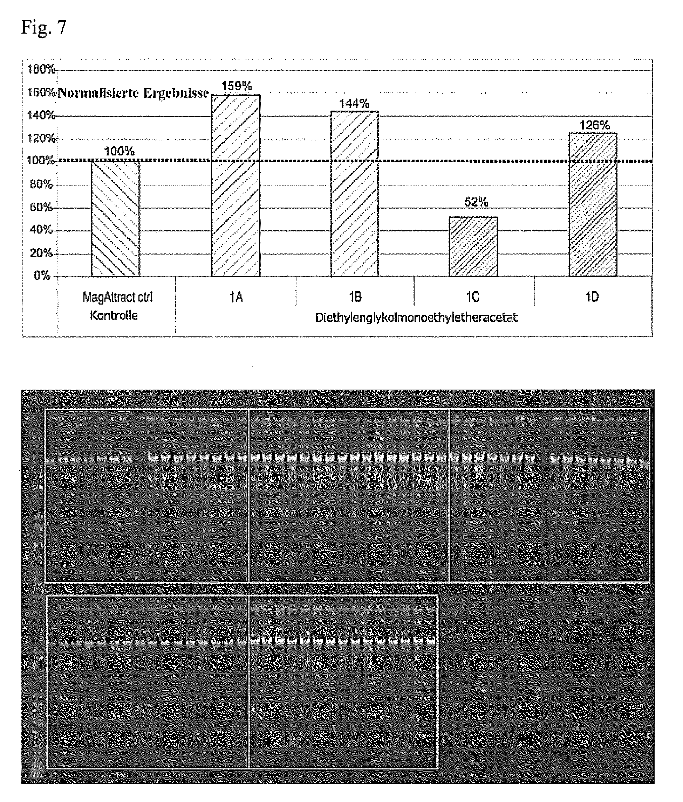

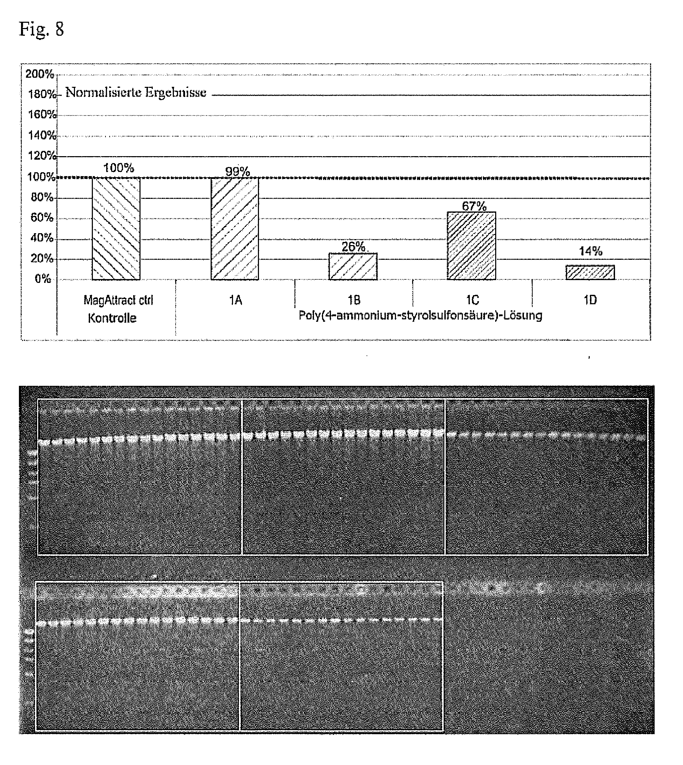

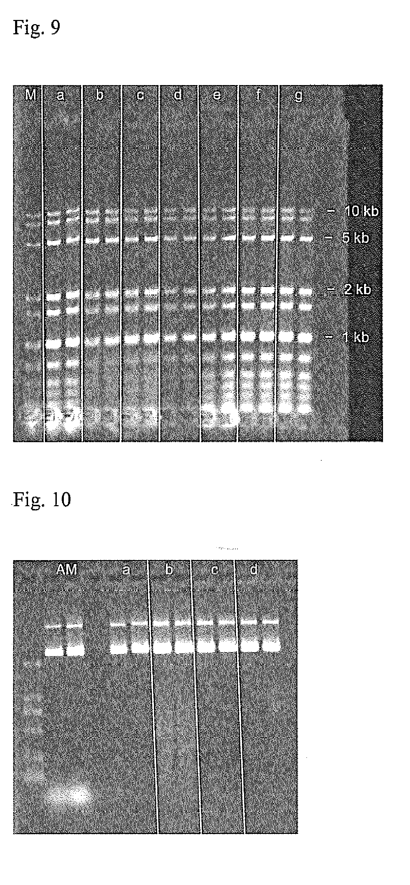

Fig. 1: Verhalten von Poly(2-ethyl-2-oxazolin) und TetraGlyme beim Einsatz im QIAamp^{®} 96 Spin Blut-Protokoll.
   Obere Tafel: mit Hilfe von β-Aktin qPCR bestimmte normalisierte Ergebnisse; untere Tafel: Agarosegel mit den individuellen Proben
   1A: 13,5% Poly(2-ethyl-2-oxazolin)
   1B: 22,5% Poly(2-ethyl-2-oxazolin); 24% Ethanol
   2A: 98,0% TetraGlyme
   2B: 73,5% TetraGlyme
Fig. 2: Verhalten von Diethylenglycolmonoethylether beim Einsatz im QIAamp^{®} 96 Spin Blut-Protokoll.
   obere Tafel: mit Hilfe von β-Actin qPCR bestimmte normalisierte Ergebnisse; untere Tafel: Agarosegel mit den individuellen Proben
   A: 99,0% Diethylenglycolmonoethylether
   B: 74,3% Diethylenglycolmonoethylether; 24% Ethanol
   C: 61,9% Diethylenglycolmonoethylether; 24% Ethanol
   D: 80,0% Diethylenglycolmonoethylether; 16% Ethanol
Fig. 3: Fig 3: Verhalten von Diethylenglycolmonoethyletheracetat beim Einsatz im QIAamp^{®} 96 Spin Blut-Protokoll.
   Tafel: mit Hilfe von β-Aktin qPCR bestimmte normalisierte Ergebnisse
   A: 99,0% Diethylenglycolmonoethyletheracetat
   B: 74,3% Diethylenglycolmonoethyletheracetat; 24% Ethanol
   C: 61,9% Diethylenglycolmonoethyletheracetat; 24% Ethanol
   D: 80,0% Diethylenglycolmonoethyletheracetat; 16% Ethanol
Fig. 4: Verhalten der Poly(4-ammonium-styrolsulfonsäure)-Lösung beim Einsatz im QIAamp^{®} 96 Spin Blut-Protokoll.
   obere Tafel: mit Hilfe von β-Aktin qPCR bestimmte normalisierte Ergebnisse; untere Tafel: Agarosegel mit den individuellen Proben
   A: 12% Poly(4-ammonium-styrolsulfonsäure)-Lösung
   B: 10% Poly(4-ammonium-styrolsulfonsäure)-Lösung
   C: 12% Poly(4-ammonium-styrolsulfonsäure)-Lösung
   D: 10% Poly(4-ammonium-styrolsulfonsäure)-Lösung
Fig. 5: Verhalten von Poly(2-ethyl-2-oxazolin) und TetraGlyme beim Einsatz im BioSprint^{®} 96 DNA Blut-Protokoll.
   obere Tafel: mit Hilfe von β-Aktin qPCR bestimmte normalisierte Ergebnisse; untere Tafel: Agarosegel mit den individuellen Proben
   1A: 13,5% Poly(2-ethyl-2-oxazolin)
   1B: 22,5% Poly(2-ethyl-2-oxazolin); 24% Ethanol
   2A: 98,0% TetraGlyme
   2B: 73,5% TetraGlyme
Fig. 6: Verhalten von Diethylenglycolmonoethylether beim Einsatz im BioSprint^{®} 96 DNA Blut-Protokoll.
   Obere Tafel: mit Hilfe von β-Aktin qPCR bestimmte normalisierte Ergebnisse; untere Tafel: Agarosegel mit den individuellen Proben
   A: 99,0% Diethylenglycolmonoethylether
   B: 74,3% Diethylenglycolmonoethylether; 24% Ethanol
   C: 61,9% Diethylenglycolmonoethylether; 24% Ethanol
   D: 80,0% Diethylenglycolmonoethylether; 16% Ethanol
Fig. 7: Verhalten von Diethylenglycolmonoethyletheracetat beim Einsatz im BioSprint^{®} 96 DNA Blut-Protokoll.
   Obere Tafel: mit Hilfe von β-Aktin qPCR bestimmte normalisierte Ergebnisse; untere Tafel:
   Agarosegel mit den individuellen Proben
   A: 99,0% Diethylenglycolmonoethyletheracetat
   B: 74,3% Diethylenglycolmonoethyletheracetat; 24% Ethanol
   C: 61,9% Diethylenglycolmonoethyletheracetat; 24% Ethanol
   D: 80,0% Diethylenglycolmonoethyletheracetat; 16% Ethanol
Fig. 8: Verhalten von Diethylenglycolmonoethyletheracetat beim Einsatz im BioSprint^{®} 96 DNA Blut-Protokoll.
   Obere Tafel: mit Hilfe von β-Aktin qPCR bestimmte normalisierte Ergebnisse; untere Tafel: Agarosegel mit den individuellen Proben
   A: 12% Poly(4-ammonium-styrolsulfonsäure)-Lösung
   B: 8% Poly(4-ammomum-styrolsulfonsäure)-Lösung
   C: 12% Poly(4-ammonium-styrolsulfonsäure)-Lösung
   D: 8% Poly(4-ammonium-styrolsulfonsäure)-Lösung
Fig. 9: QIAquick^{®} Protokoll und die daraus resultierende Reinigung der Gel Pilot 1kb Ladder. Die erste Spur stellt den ungereinigten Marker dar, die Spur "a" enthält ein QIAquick-gereinigtes Fragment, welches hier als Referenz verwendet wird. Unter den genannten Bedingungen sind keine signifikanten Verluste in Bezug auf die Ergebnisse und/oder die größenabhängige Reinigung beobachtet worden.
   M) unbehandelte "Gel Pilot^{®} 1kb Ladder"
   a) Puffer PM;
   b) 5M GuHCl, 100mM Na-Ac, 12% Poly(4-ammonium-styrolsulfonsäure)
   c) 5M GuHCl, 100mM Na-Ac, 12% Poly(4-ammonium-styrolsulfonsäure); 20% Isopropanol
   d) 5M GuHCl, 100mM Na-Ac, 13,5 %Poly(2-ethyl-2-oxazolin)
   e) 5M GuHCl, 100mM Na-Ac, 10% Poly(2-ethyl-2-oxazolin); 20% Ethanol
   f) 5M GuHCl, 100mM Na-Ac, 30% TetraGlyme
   g) 5M GuHCl, 10mM Tris pH 7,5, 30% TetraGlyme
Fig. 10: QIAquick-Reinigung einer Mischung aus Plasmid-DNA und Oligonukleotiden. Die Oligonukleotide werden durch die alternativen Reinigungs-Protokolle entfernt.
   AM) Ausgangsmaterial: Gemisch aus Plasmid DNA und einem DNA Oligonukleotid

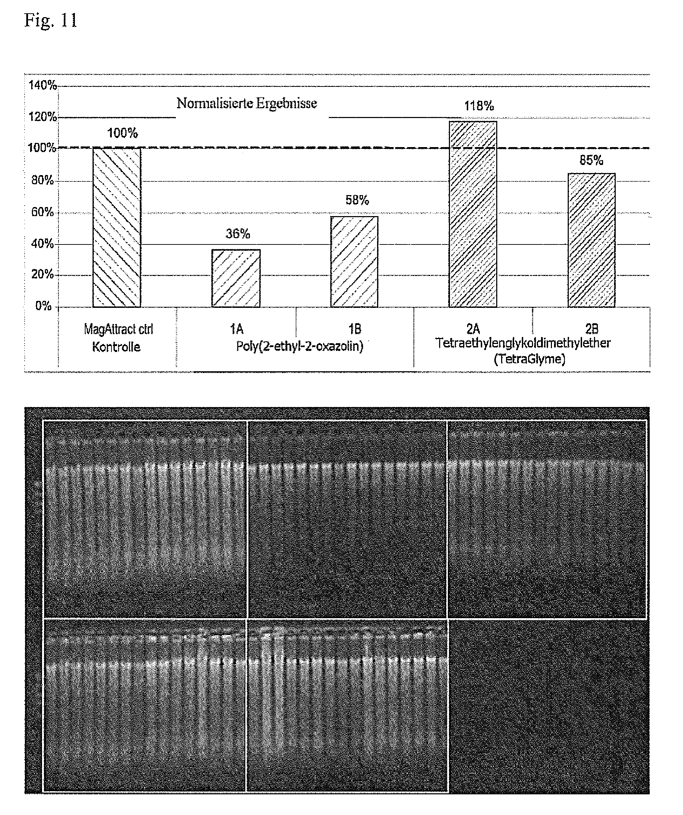

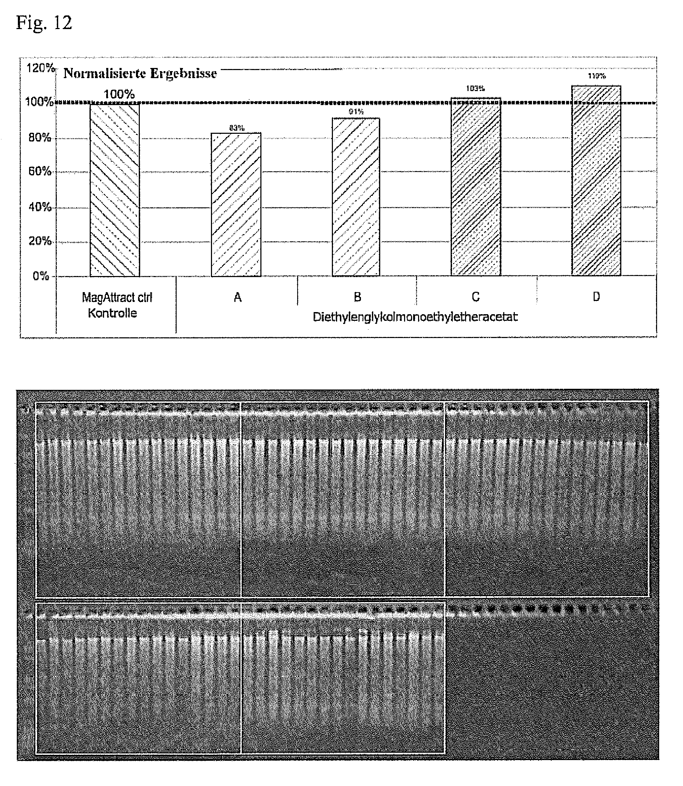

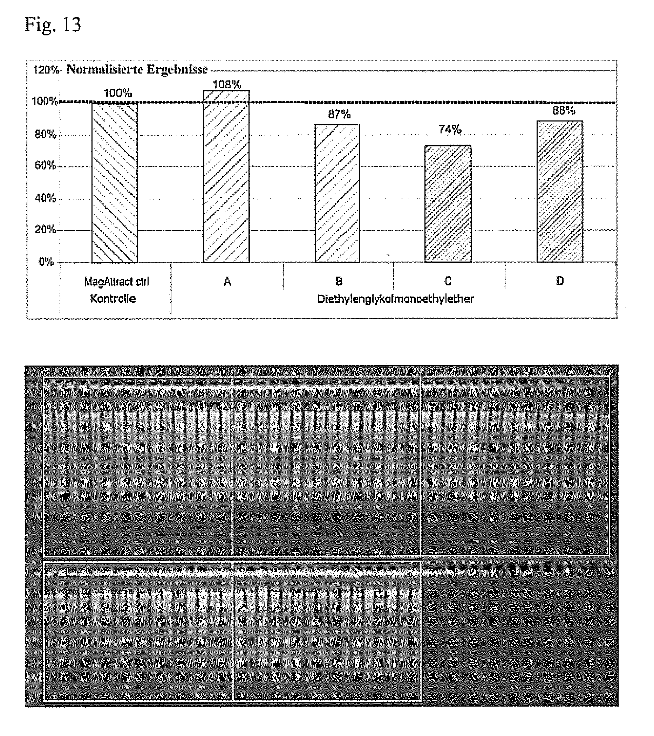

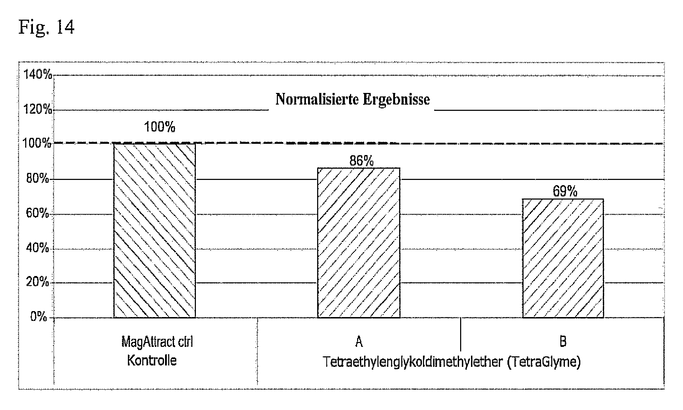

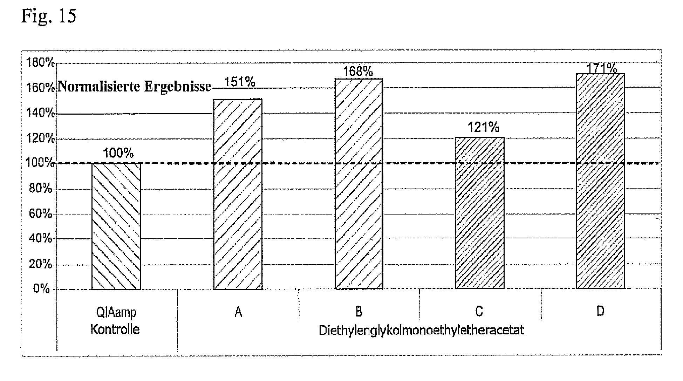

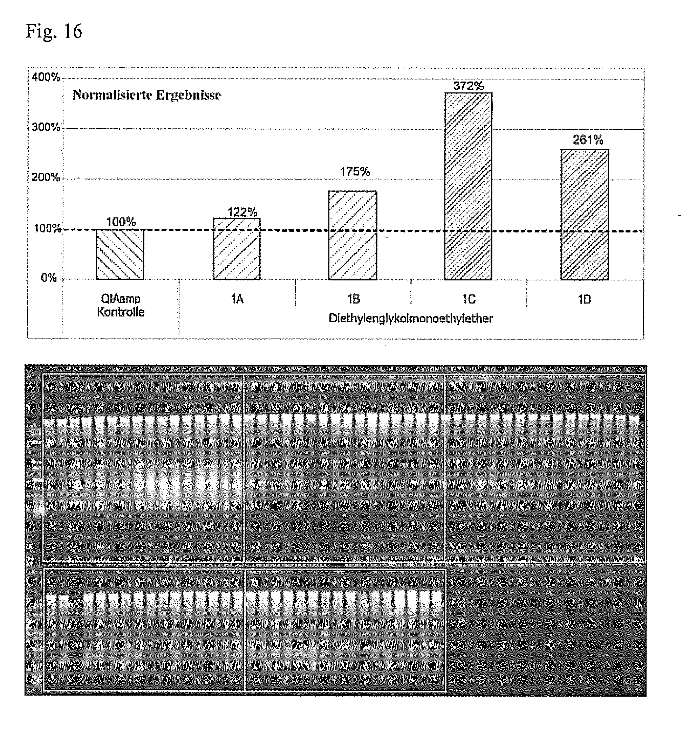

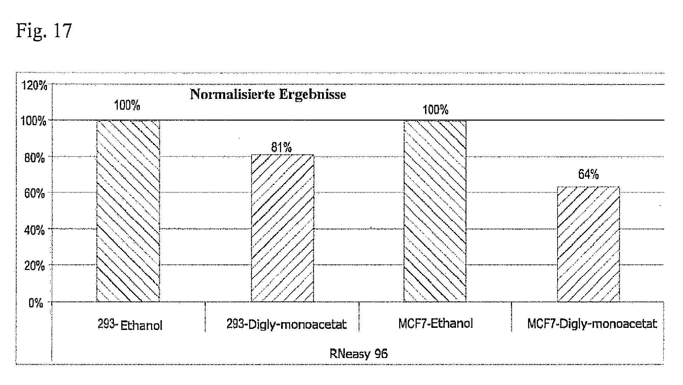

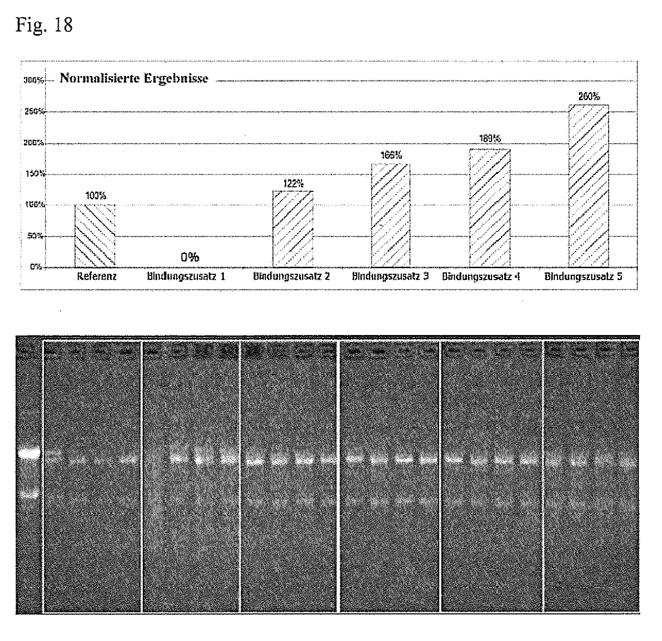

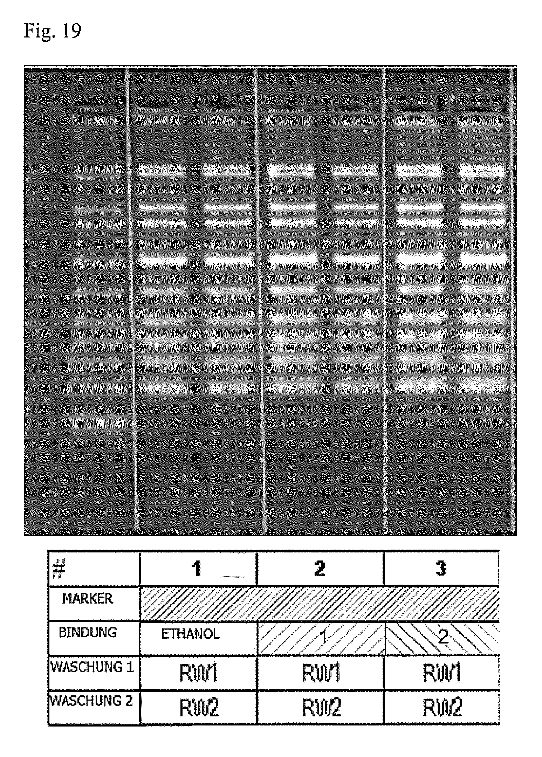

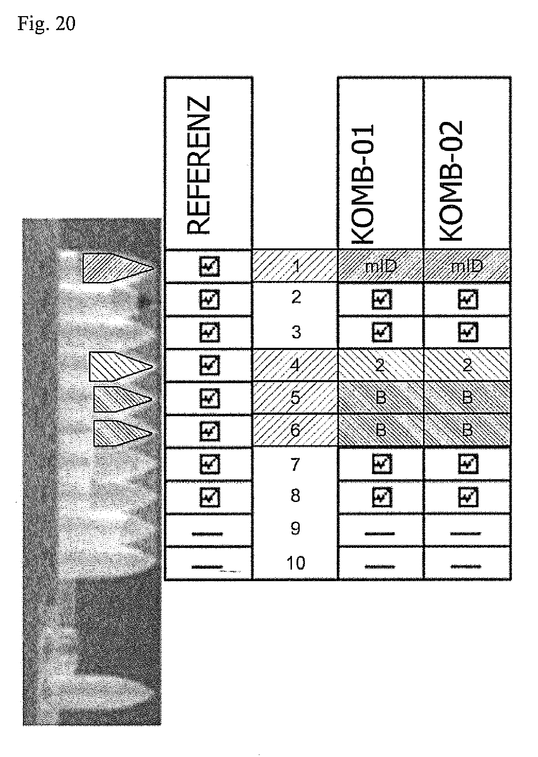

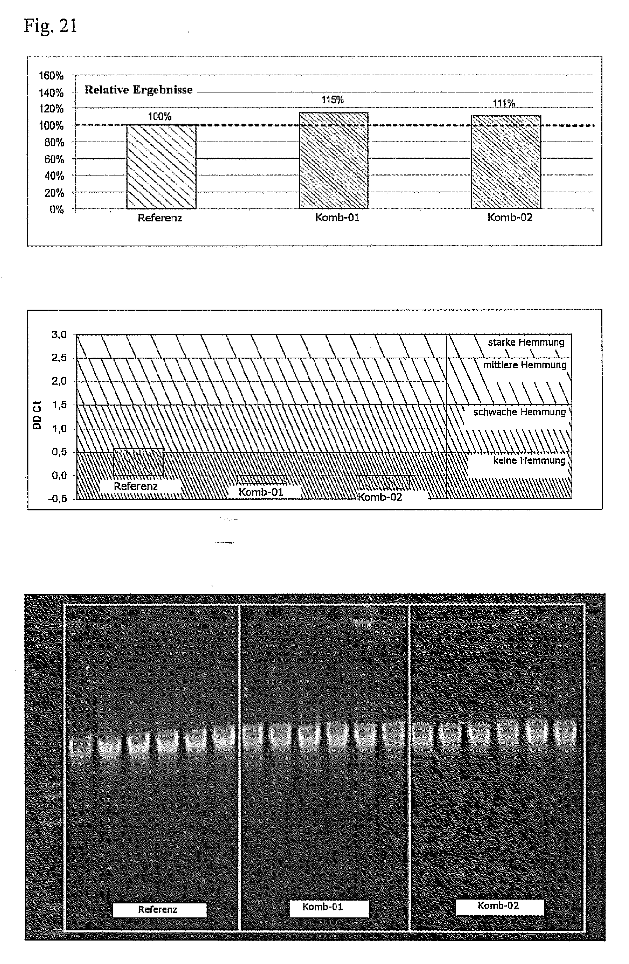

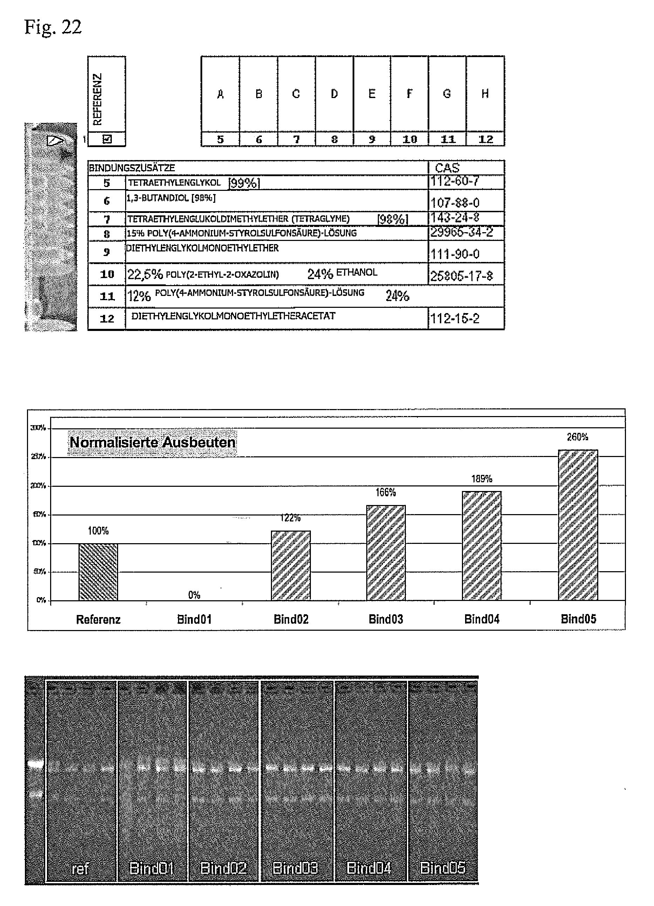

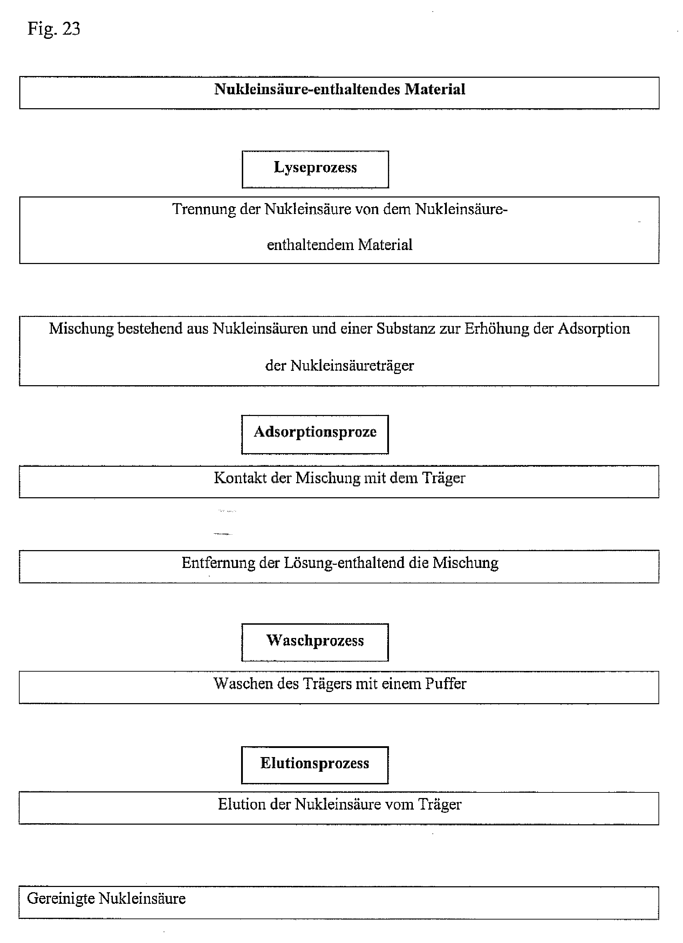

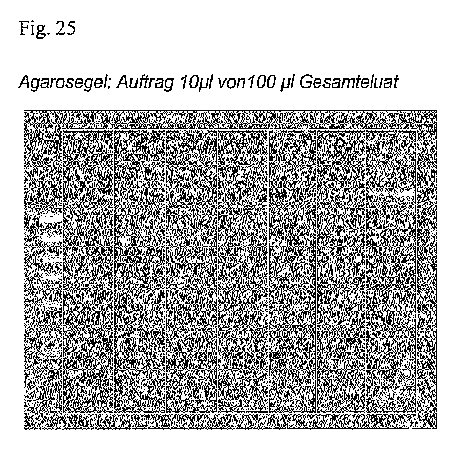

   a) Puffer PM
   b) 5M GuHCl, 100mM Na-Ac, 12% Poly(4-ammonium-styrolsulfonsäure)
   c) 5M GuHCl, 100mM Na-Ac, 30% TetraGlyme
   d) 5M GuHCl, 10mM Tris pH 7,5, 30% TetraGlyme
Fig. 11: Verhalten von Poly(2-ethyl-2-oxazolin) und TetraGlyme beim Einsatz im BioSprint^{®} 96 Gewebe-Protokoll.
   Obere Tafel: mit Hilfe von Mouse-GapDH qPCR bestimmte normalisierte Ergebnisse; untere Tafel: Agarosegel
   1A: 13,5% Poly(2-etliyl-2-oxazolin)
   1B: 22,5% Poly(2-ethyl-2-oxazolin); 24% Ethanol
   2A: 98,0% TetraGlyme
   2B: 73,5% TetraGlyme; 24% Ethanol
Fig. 12: Verhalten von Diethylenglycolmonoethyletheracetat beim Einsatz im BioSprint^{®} 96 Gewebe-Protokoll.
   Obere Tafel: mit Hilfe von Mouse-GapDH qPCR bestimmte normalisierte Ergebnisse; untere Tafel: Agarosegel
   A: 99,0% Diethylenglycolmonoethyletheracetat
   B: 74,3% Diethylenglycolmonoethyletheracetat; 24% Ethanol
   C: 61,9% Diethylenglycolmonoethyletheracetat; 24% Ethanol
   D: 80,0% Diethylenglycolmonoethyletheracetat; 16% Ethanol
Fig. 13: Verhalten von Diethylenglycolmonoethylether beim Einsatz im BioSprint^{®} 96 Gewebe-Protokoll.
   Obere Tafel: mit Hilfe von Mouse-GapDH qPCR bestimmte normalisierte Ergebnisse; untere Tafel: Agarosegel
   A: 99,0% Diethylenglycolmonoethylether
   B: 74,3% Diethylenglycolmonoethylether; 24% Ethanol
   C: 61,9% Diethylenglycolmonoethylether; 24% Ethanol
   D: 80,0% Diethylenglycolmonoethylether; 16% Ethanol
Fig. 14: Verhalten von TetraGlyme beim Einsatz im DNeasy^{®} 96 Gewebe-Protokoll
   Tafel: mit Hilfe von Mouse-GapDH qPCR bestimmte normalisierte Ergebnisse
   A: 98,0% TetraGlyme
   B: 73,5% TetraGlyme
Fig. 15: Verhalten von Diethylenglycolmonoethyletheracetat beim Einsatz im DNeasy^{®} 96 Gewebe-Protokoll.
   Tafel: mit Hilfe von Mouse-GapDH qPCR bestimmte normalisierte Ergebnisse
   A: 99,0% Diethylenglycolmonoethyletheracetat
   B: 74,3% Diethylenglycolmonoethyletheracetat; 24% Ethanol
   C: 61,9% Diethylenglycolmonoethyletheracetat; 24% Ethanol
   D: 80,0% Diethylenglycolmonoethyletheracetat; 16% Ethanol
Fig. 16: Verhalten von Diethylenglycolmonoethyletheracetat beim Einsatz im DNeasy^{®} 96 Gewebe-Protokoll.
   Obere Tafel: mit Hilfe von Mouse-GapDH qPCR bestimmte normalisierte Ergebnisse; untere Tafel: Agarosegel
   A: 99,0% Diethylenglycolmonoethylether
   B: 74,3% Diethylenglycolmonoethylether; 24% Ethanol
   C: 61,9% Diethylenglycolmonoethylether; 24% Ethanol
   D: 80,0% Diethylenglycolmonoethyletheracetat; 16% Ethanol
Fig. 17: Verhalten von Diethylen-glycolmonoethyletheracetat beim Einsatz im DNeasy^{®} 96 Gewebe-Protokoll.
   Tafel: mit Hilfe von Lamin RT-qPCR bestimmte normalisierte Ergebnisse; die verwendeten Zellen waren "293" und MCF7
Fig. 18: Verhalten von verschiedenen Ersatzchemikalien für Ethanol beim Einsatz im DNeasy^{®} 96 Protokoll.
   Obere Tafel: mit Hilfe von Mouse-GapDH qPCR bestimmte normalisierte Ergebnisse; untere Tafel: Agarosegel
   Bindungszusatz 01 = 12% Poly(4-ammonium-styrolsulfonsäure)-Lösung (versagte bei PCR)
   Bindungszusatz 02 = 98% TetraGlyme
   Bindungszusatz 03 = 73,5% TetraGlyme; 24% Ethanol
   Bindungszusatz 04 = 99% Diethylenglycolmonoethyletheracetat
   Bindungszusatz 05 = 80% Diethylenglycolmonoethyletheracetat; 16% Ethanol
Fig. 19: Experiment zur Fragmentgrößen-Inhibition
   RNeasy^{®} hemmt während der Reinigung kleine RNAs (5,8 S; tRNA; miRNA; ...). Die Ausschlussgröße beträgt ca. 150 Basisgrößen. In diesem Experiment wird demonstriert, dass die Größeninhibition der Testchemikalien mit den Referenzwerten von Ethanol vergleichbar ist.
   Bindungszusatz 1 = 98% TetraGlyme;
   Bindungszusatz 2 = 80% Diethylenglycolmonoethyletheracetat; 16% Ethanol
Fig. 20: Kassetteneinstellung der EZ1^{®} DNA Blut 200µl Reagenzkassette Puffer ML auf Postion 1
   m1D: 4,5 M GTC; 50 mM NH4Cl; 45 mM Tris pH 7,5; 20 mM EDTA; 2.0% Triton-X-100
   m19: 4,5 M GTC; 1,0 M NaCl; 50 mM NH4Cl; 45 mM Tris pH 7,5; 20 mM EDTA; 2.0% Triton-X-100
   MW1- Ersatz-Puffer auf Postion 4
      "2": 49% 1,3- Butandiol ; 2.5 MGuHCl
   MW2-Ersatz-Puffer auf Postion 5+6
      "B": 60% 1,3 Butandiol ; 100mM NaCl; 10mM Tris-Cl pH 7.5
Fig. 21: Verhalten von verschiedenen Ersatzchemikalien für Ethanol beim Einsatz im EZ1^{®} DNA Blut 200ul Protokoll.
   obere linke Tafel: mit Hilfe von β-Aktin qPCR bestimmte normalisierte Ergebnisse; rechte Tafel: "Delta-Delta-CT"-Analyse von verschiedenen Proben-Ausgangsmengen. Beim Rechenvorgang wird die gemessene Delta-CT mit der theoretischen Delta-CT verglichen, wodurch der Zahlenwert des PCR-Inhibitions-Grades offenbart wird; untere Tafel: Agarosegel
Fig. 22: Verhalten von verschiedenen Ersatzchemikalien für Ethanol beim Einsatz im ersten Bindungsschritt des EZ1^{®} - RNA- Protokolls.
   Obere Tafel: Kassetteneinstellung; der EZ1^{®} DNA Blut 200µl Reagenzkassette; mittlere Tafel: mit Hilfe von MapK2 RT qPCR bestimmte normalisierte Ergebnisse; untere Tafel: Agarosegel
   Bind01 = 12% Poly(4-ammonium-styrolsulfonsäure) Lösung (versagte bei RT qPCR)
   Bind02 = 98% TetraGlyme
   Bind03 = 73,5% TetraGlyme; 24% Ethanol
   Bind04 = 99% Diethylenglycolmonoethyletheracetat
   Bind05 = 80% Diethylenglycolmonoethyletheracetat; 16% Ethanol
Fig. 23: zeigt das erfindungsgemäße Verfahren in einer möglichen Ausführungsform.

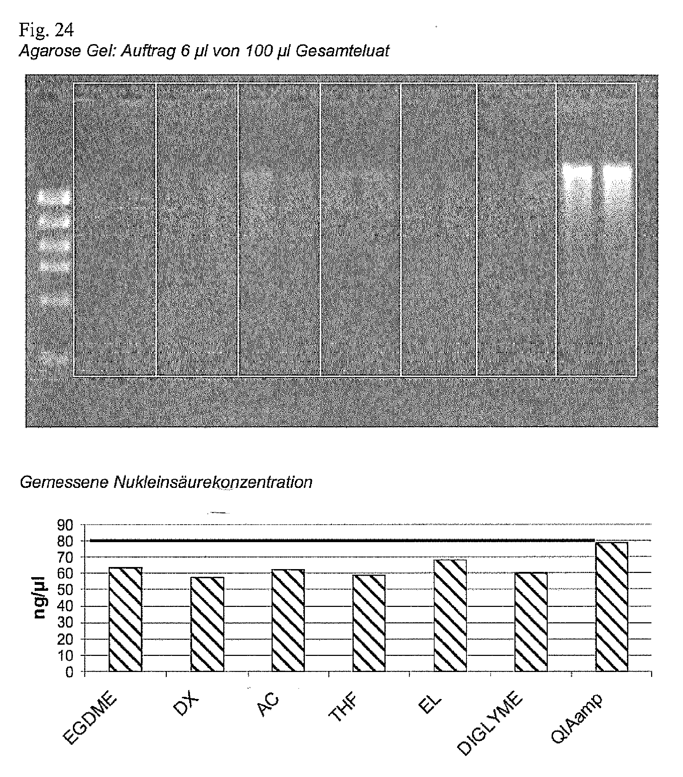

Fig. 24: Vergleich der in US 2004/167324 A1 verwendeten Bindungszusätze mit den Methoden des Stands der Technik am Beispiel des QIAamp^{®} Blut Protokolls.
   1: EGDME (Ethylenglycoldimethylether); 2: DX (1,4-Dioxan); 3: AC (Aceton); 4: THF (Tetrahydrofuran); 5: EL (Ethyllactat); 6: DIGLYME (Diethylenglycoldimethylether); 7: Referenz - MagAttract^{®} Blut Protokoll
Fig. 25: Vergleich der in US 2004/167324 A1 verwendeten Bindungszusätze mit den Methoden des Stands der Technik am Beispiel des MagAttract^{®} Blut Protokolls.
   1: EGDME (Etlrylenglycoldimethylether); 2: DX (1,4-Dioxan); 3: AC (Aceton); 4: THF (Tetrahydrofuran); 5: EL (Ethyllactat); 6: DIGLYME (Diethylenglycoldimethylether); 7: Referenz - MagAttract^{®} Blut Protokoll

**Tabelle 1 kommerziell erhältliche Produkte der Fa. QIAGEN, im Rahmen der Beispiele eingesetzt**

| | |
|---|---|
| MagAttract^{®} suspension G | Suspension mit Magnetpartikeln |
| Puffer PE | Waschpuffer mit schwacher organischer Base |
| Puffer AE | Niedrigsalz-Puffer |
| Puffer EB | Wässriger Elutionspuffer |
| Puffer TE | Elutionspuffer; 10mM TrisCL1, mM EDTH pH8 |
| RNase-free water | Reinstwasser, RNase frei |
| Puffer AL | Lysepuffer enthaltend Guanidiniumhydrochlorid |
| Puffer RLT | Puffer umfassend Thiocyanat |
| Puffer ATL | Puffer enthaltend EDTA und SDS |
| Puffer ML | Puffer enthaltend Guanidiniumthiocyanat und t-Octylphenoxypolyoxyethanol |
| Puffer AP1 | Puffer enthaltend EDTA und SDS |
| Puffer AW1 | Waschpuffer enthaltend Guanidiniumhydrochlorid |
| Puffer AW2 | Waschpuffer enthaltend Natriumazid |
| Puffer RW1 | Alkoholhaltiger Puffer mit Guanidiniumsalz |
| Puffer RPE | Wässriger Puffer |
| ProtK | Proteinkinase K |
| Puffer PM | Bindepuffer enthaltend Guanidiniumchlorid und 2-Propanol |
| Puffer MW1 Ethanol | Einsatzpuffer enthaltend Guanidimumhydrochlorid und Ethanol |
| Puffer MW2 Ethanol | Puffer mit Lithiumchlorid und Ethanol |
| GTC | Guanidiniumthiocyanat |
| MW1 Ersatz-Puffer | Einsatzpuffer enthaltend Guanidiniumlhydrochlorid |
| MW2 Ersatz-Puffer | Puffer mit Lithiumchlorid |
| RDD | RNAse-freier Puffer |
| AlAamp Spin | QiaAmp® Spin-Säulen |
| K-AC | Kaliumacetat |
| EGME | Ethylenglycolmonomethylether |
| MagSep | Magnetische Separation |
| MagStep | Schritt zur Magnetischen Separation |

Die in Tabelle 1 aufgeführten Reagenzien und Puffer sowie die hierin beschriebenen Protokolle sind Veröffentlichungen und kommerziell erhältliche Produkte der Firma QAIGEN GmbH, Hilden.

### Beispiel 1: "BioSprint^{®} 96 DNA Blut"

BioSprint^{®} 96 mit Protokoll-File: "BS96_DNA_Blut_200"
Lyse:
   - 200 µl Blut
   - 200 µl Puffer AL
   - 20 µl QIAGEN Protease
   - Inkubation in einem Thermomixer für 15 min bei 56°C bei 1400 Umdrehungen pro Minute
Bindung:
   - Zugabe von 200 µl Isopropanol zum Standard-Referenzprotokoll
   - Ersatzstoffe für Isopropanol (jeweils 200 µl hinzugeben) :
      1A) 98,0% TetraGlyme
      1B) 73,5% TetraGlyme ; 24% Ethanol
      2A) 99% Diethylenglycolmonoethyletheracetat
      2B) 74,3% Diethylenglycolmonoethyletheracetat; 24% Ethanol
      2C) 61,9% Diethylenglycolmonoethyletheracetat; 24% Ethanol
      2D) 80% Diethylenglycolmonoethyletheracetat; 16% Ethanol
      3A) 12% Poly(4-ammonium-styrolsulfbnsäure)-Lösung
   - Zugabe von 30 µl MagAttract Suspension G Waschschritte
   - 1x Puffer AW1 (650 µl)
   - 1x Puffer AW1 (500 µl)
   - 2x Puffer Aw2 (500 µl)
      Spülung mit wässriger Lösung: 0,02% Tween® 20
Elution: 200 µl Puffer TE in 96-well MicroTubePack-MicroPlate

### Beispiel 2: QIAamp^{®} 96 Spin Blut-Protokoll

Lyse
   - 200 µl Blut
   - 200 µl Puffer AL
   - 20 µl QIAGEN Protease
   - Inkubation für 15 min bei 56°C
Bindung
   • Zugabe von 200 µl Ethanol zum Standard-Referenzprotokoll
   • Ersatzstoffe für Ethanol (jeweils 200 µl hinzugeben) :
      1A) 99% Diethylenglycolmonoethylether
      1B) 74,3% Diethylenglycolmonoethylether; 24% Ethanol
      1C) 61,9% Diethylenglycolmonoethylether; 24% Ethanol
      1D) 80% Diethylenglycolmonoethylether; 16% Ethanol
      2A) 99% Diethylenglycolmonoethyletheracetat
      2B) 74,3% Diethylenglycolmonoethyletheracetat; 24% Ethanol
      2C) 61,9% Diethylenglycolmonoethyletheracetat; 24% Ethanol
      2D) 80% Diethylenglycolmonoethyletheracetat; 16% Ethanol
      3A) 98,0% TetraGlyme
      3B) 73,5% TetraGlyme; 24% Ethanol
      4) 10% Poly(4-ammonium-styrolsulfonsäure)-Lösung ; 24% Ethanol
   • im 96-well Deep-Well-Block vermischen und auf QIAamp^{®} 96 Platte übertragen Waschschritte
   • 1x Puffer AW1 (650 µl)
   • 1x Puffer AW2 (500 µl)
Elution: 200 µl Puffer TE in Elution Microtube Rack

### Beispiel 3: "BiaSprint^{®} 96 DNA Tissue"

BioSprint^{®} 96 Protokoll-File: "BS96_DNA_Blut_200"
Lyse
   - 200 µl Lysat ( 25mg Gewebe + 180 µl Puffer ATL + 20µl Proteinase K, Inkubation über Nacht bei 56°C)
   - Zugabe von 200 µl Puffer AL Bindung
   - Zugabe von 200 µl Isopropanol zum Standard-Referenzprotokoll
   - Ersatzstoffe für Isopropanol (jeweils 200 µl einzugeben)
      1A) 98,0% TetraGlyme
      1B) 73,5% TetraGlyme ; 24% Ethanol
      2A) 99% Diethylenglycolmonoethyletheracetat
      2B) 74,3% Diethylenglycolmonoethyletheracetat; 24% Ethanol
      2C) 61,9% Diethylenglycolmonoethyletheracetat; 24% Ethanol
      2D) 80% Diethylenglycolmonoethyletheracetat; 16% Ethanol
      3A) 99% Diethylenglycolmonoethylether
      3B) 74,3%-Biethylenglycolmonoethylether; 24% Ethanol
      3C) 61,9% Diethylenglycolmonoethylether; 24% Ethanol
      3D) 80% Diethylenglycolmonoethylether; 16% Ethanol
   - + 30 µl MagAttract Suspension G Waschschritte
   - 1x Puffer AW1 (650 µl)
   - 1x Puffer AW1 (500 µl)
   - 2x Puffer AW2 (500 µl)
   - Spülung mit wässriger Lösung: 0,02% Tween 20 (500 µl)
Elution: 200 µl Puffer TE in Microtube-Platte

### Beispiel 4: "DNeasy^{®} 96 Tissue"

Lyse
   - 200 µl Lysat (25mg Gewebe + 180 µl Puffer ATL + 20µl Proteinase K, Inkubation über Nacht bei 56°C)
   - Zugabe von 200 µl Puffer AL Bindung
   - Zugabe von 200 µl Ethanol zum Standard-Referenzprotokoll
   - Ersatzstoffe für Ethanol (jeweils 200 µl zugeben) :
      1A) 99% Diethylenglycolmonoethylether
      1B) 74,3% Diethylenglycolmonoethylether; 24% Ethanol
      1C) 61,9% Diethylenglycolmonoethylether; 24% Ethanol
      1D) 80% Diethylenglycolmonoethyletheracetat; 16% Ethanol
      2A) 99% Diethylenglycolmonoethyletheracetat
      2B) 74,3% Diethylenglycolmonoethyletheracetat; 24% Ethanol
      2C) 61,9% Diethylenglycolmonoethyletheracetat; 24% Ethanol
      2D) 80% Diethylenglycolmonoethyletheracetat; 16% Ethanol
      3A) 98,0% TetraGlyme
      3B) 73,5% TetraGlyme; 24% Ethanol
   - im 96-well Deep-Well-Block vermischen und auf DNeasy^{®} 96 Platte übertragen Waschschritte
   - 1x Puffer AW1 (650 µl)
   - 1x Puffer AW2 (500 µl)
Elution: 200 µl Puffer TE in Elution Microtube Rack

### Beispiel 5: RNeasy^{®} 96

Bindung
   - 350 µl Puffer RLT - Lysat ( "293" Zellen; 2x105 Zellen/Probe)
   - Zugabe von 350 µl Ethanol zum Standard-Referenzprotokoll
   - Ersatzstoffe für Ethanol (jeweils 200 µl zugeben) :
      1) 80% Diethylenglycolmonoethyletheracetat; 16% Ethanol
      2) 98% TetraGlyme
   - im S-Block vermischen und auf die RNeasy^{®} 96 Platte übertragen Waschschritte
   - 2x Puffer RW1 (650 µl)
   - 2x Puffer RPE (500 µl)
Elution: 100 µl RNase-freies Wasser in Elution Microtube Rack

### Beispiel 6: QIAquick^{®}

Bindung
   - 1 Volumen Nukleinsäure-haltige Probe
   - + 5 Volumen Puffer PM (Standard-Referenzprotokoll)
   - Substitute für Puffer PM
      ○ 12% Poly(4-ammonium-styrolsulfonsäure); 5M GuHCl; 100mM Na-Acetat
      ○ 30% TetraGlyme; 5M GuHCl; 10mM Tris pH 7.5
      ○ 10% Poly(2-ethyl-2-oxazoline), 5M GuHCl, 100mM Na-Acetat; 20% Ethanol
   - Laden der QIAamp^{®} MinElute Spincolumn; Zentrifugation für 1 min bei 8000 Umdrehungen pro Minute
Waschschritte
   • 1 Waschschritt mit Puffer PE
   • "Dry Spin"
Elution: 40 µl RNase-freies Wasser in Elution Microtube

### Beispiel 7: EZ1^{®} DNA Blut 200 µl Protokoll

EZ1^{®} DNA Blut 200µl Reagenzkassette - Inhaltsplan

| Füllposition | Inhalt | Menge (µl) | Austauschposition |
|---|---|---|---|
| 1 | Lysepuffer (Puffer ML) | 740 | ⇐ |
| 2 | "MagAttract Suspension B" | 300 | |
| 3 | "Bead puffer" | 60 | |
| 4 | Waschpuffer I (Puffer MW1 Ethanol) | 900 | ⇐ |
| 5 | Waschpuffer II (Puffer MW2 Ethanol) | 900 | ⇐ |
| 6 | Waschpuffer II (Puffer MW2 Ethanol) | 900 | ⇐ |
| 7 | Spülung (Reinstwasser) | 1000 | |
| 8 | Eluierungs-Puffer (Reinstwasser) | 220 | |
| 9 | leer | 0 | |
| 10 | leer | 1000 | |

Ersatz-Puffer

| ML-Ersatz-Puffer | Position |
|---|---|
| 4,5 M GTC; 1,0 M NaCl; 50 mM NH₄CL; 45 mM Tris pH 7,5; 20 mM EDTA; 2.0% Triton-X-100 | 1 |
| 4,5 M GTC; 50 mM NH₄Cl; 45 mM Tris pH 7,5; 20 mM EDTA; 2.0% Triton-X-100 | 1 |
| MW1 Ersatzpuffer | Position |
| 49% 1,3-Butandiol; 2.5 M GuHCl | 4 |
| MW2 Ersatzpuffer | Position |
| 60% 1,3 Butandiol; 100 mM NaCl; 10 mM Tris-Cl pH 7,5 | 4+6 |

### Beispiel 8: EZ1^{®} - RNA Protokoll

EZ1^{®} RNA Reagenzkassette - Inhaltsplan

| Füllposition: | Inhalt | Menge (µl) | Austauschp osition |
|---|---|---|---|
| 1 | Puffer RPE + 96% EtOH (= Puffer RPE Arbeitslösung) | 400 + 100 | ⇐ |
| 2 | 0,5M LiCL + "MagAttract Suspension B" | 320 + 80 | |
| 3 | Puffer MW1 + 96% EtOH (= Puffer AW 1 Arbeitslösung) | 344 + 456 | ⇐ |
| 4 | Puffer RPE + 96% EtOH (= Puffer RPE Arbeitslösung) | 160 + 640 | ⇐ |
| 5 | Puffer RDD | 245 | |
| 6 | Puffer MW 1 (= Puffer AW1-Konzentrat) | 250 | |
| 7 | Puffer MW1 + 96% EtOH (= Puffer AW1 ⇐ | 251 + 785 | ⇐ |
| | Arbeitslösung 2) | | |
| 8 | Puffer RPE + 96% EtOH | 180+270 | ⇐ |
| 9 | Reinstwasser | 1000 | |
| 10 | Reinstwasser | 200 | |

Ersatz-Puffer

| Bindungszusätze | Kasettenposition |
|---|---|
| Tetraethylenglycol (99%) | 1 |
| 1,3-Butandiol (98%) | 1 |
| 80% Diethylenglycolmonoethyletheracetat; 16% Ethanol | 1 |

| Waschpuffer | |
|---|---|
| 56% 1,3-Butandiol; 3 M GuHCl | 3 |
| 60% 1,3 Butandiol; 100mM NaCl; 10mM Tris-Cl pH 7,5 | 4 |
| 65% Tetraethylenglycol; 900 mM GTC; 10 mM Tris/CL pH 7,5 | 7 |
| 60% 1,3 Butandiol; 30mM NaCl; 10mM Tris-Cl pH 7,5 | 8 |

### Beispiel 9: Vergleich der erfindungsgemäßen Bindungszusätze mit denen des Standes der Technik

Gegenstand: Vergleich der in US 2004/167324 A1 (Hitachi) verwendeten organischen Lösungsmittel als Bindungszusätze für klassische chaotrope Bindungen auf Siliziumdioxid mit den erfindungsgemäß verwendeten Referenzbindungszusätzen für QlAamp^{®} und MagAttract^{®}.

### Material:

Blut und Puffer
Lysepuffer: 3 M GuHcl; 5% Triton X-100
Waschpuffer: 25 mM K-Acetat; 50% Ethanol
Elutionspuffer: Puffer TE

**Substitutionsreagenzien**

| | |
|---|---|
| 1 EGME | Ethylenglycoldimethylether |
| 2 DX | 1,4-Dioxan |
| 3 AC | Aceton |
| 4 THF | Tetrahydrofuran |
| 5 EL | Ethyllaktat |
| 6 DIGLYME | Diethylenglycoldimethylether |

### Methode: Präparation genomischer DNA aus 100 µl Blut mittels QIAamp® Spin Columns:

1. 100µl Blut + 10µl Proteinase K + 100µl Lysepuffer
2. Vermischung und Inkubation für 10min bei 56°C
3. Zugabe von 100µl Substitutionsreagenz und Vermischung
4. Beladung des Lysats auf die QIAamp^{®} Spin Säule; Zentrifugation für 30 sec bei 8000 Umdrehungen pro Minute
5. Waschen mit 3x 500µl Waschpuffer; Zentrifugation jeweils für 30 sec bei 8000 Umdrehungen pro Minute
6. "Dry-spin" für 1 min bei 14.000 Umdrehungen pro Minute
7. Zugabe von 100 µl Elutionsspuffer, 2 min lang warten und für max. 1 min in neuem Sammelröhrchen durch Zentrifugation eluieren

Kontrolle: Q1Aamp^{®} Blut Mini mit 100µl Blut durchgeführt und mit 100µl TE eluiert

### Ergebnisse

UV-Quantifizierung

| | Additiv | OD260 | Mittelw. | Konz ng/µl | Färbung Membran |
|---|---|---|---|---|---|
| 1 | EGDME | 0,266 | 0,254 | 63,38 | ++ |
| | | 0,241 | | | |
| 2 | DX | 0,229 | 0,231 | 57,63 | ++ |
| | | 0,232 | | | |
| 3 | AC | 0,244 | 0,248 | 62,00 | ++ |
| | | 0,252 | | | |
| 4 | THF | 0,24 | 0,235 | 58,75 | - |
| | | 0,23 | | | |
| 5 | EL | 0,266 | 0,274 | 68,50 | + |
| | | 0,282 | | | |
| 6 | DIGLYME | 0,24 | 0,241 | 60,25 | + |
| | | 0,242 | | | |
| 7 | QIAamp^{®} | 0,31 | 0,316 | 78,88 | - |
| | | 0,321 | | | |

Zeichenerklärung Membranfärbung: ++: stark gefärbt; +: leicht gefärbt; -: keine Färbung

Die Ergebnisse des Vergleichs sind in Fig. 24 dargestellt.
Beim gegebenen Aufbau haben die organischen Lösungsmittel, die in US 2004/167324 A1 zum Einsatz kamen, als Zusätze von DNA an Siliziumdioxid-Membranen versagt. Auf dem Agarosegel sich sehr niedrige Ausbeuten zu erkennen während die UV OD-Messungen auf eine Überquantifizierung hinweisen.

### Methode: Präparation genomische DNA aus 100 µl Blut mittels magnetischen Silicapartikeln:

MagBead® Prozedur
1. 200 µl Blut + 20 µl Proteinase K + 200 µl Lysepuffer
2. Mischen und bei 56°C 10 min inkubieren
3. 215 µl Substitutionsreagenz und 30 µl MagAttract Suspension A hinzugeben
4. Im Thermomixer schütteln; 5 min bei 800 rpm; eingangs kurzes Mischen auf dem Voriexmischer
5. Magnetseparation auf einer geeigneten Vorrichtung und entfernen des Überstandes
6. Waschen mit 3 x 5000 µl Waschpuffer
7. Lufttrocknen der Magnetpartikel
8. Elution: 100 µl Puffer TE; 1 min mischen und Magnetseparation. Überstand enthält die präparierte genomische DNA und wird in ein geeignetes Gefäß überführt.

Referenzmethode: MagAttract^{®} Blut
1. 200 µl Blut + 20 µl QIAGEN-Protease + 200 µl Puffer AL
2. Mischen und inkubieren (10 min bei 56°C)
3. Zugabe von 200 µl Isopropanol und 30 µl MagAttract® Suspension A
4. Im Thermomixer schütteln; 5 min bei 800 rpm; eingangs kurzes Mischen auf dem Vortexmischer
5. Magnetseparation auf einer geeigneten Vorrichtung und entfernen des Überstandes
6. Waschen mit 500 µl Puffer AW1 und 500 µl Puffer AW2
7. Lufttrocknen der Magnetpartikel
8. Elution: 100 µl Puffer TE; 1 min mischen und Magnetseparation. Überstand enthält die präparierte genomische DNA und wird in ein geeignetes Gefäß überführt.

Wie das Agarosegel in Figur 25 zeigt, sind die Ausbeuten an genomischer DNA für die Proben, die mittel magnetischen Silicapartikeln und den in US 2004/167324 A1 verwendeten organischen Lösungsmitteln als Bindungszusätze präpariert wurden, sehr gering. Die beobachteten geringen Ausbeuten nach "US 2004/167324 A1" sind unabhängig von der Beschaffenheit des adsorptiven Mediums (magnetische Siliziumdioxid-Partikel oder Siliziumdioxid-Membranen).

## Patentansprüche

1. Verfahren zur Extraktion von Nukleinsäuren aus einer Lösung umfassend die Schritte:
(a) Hinzufügen eines Bindungsmediators zu der Nukleinsäure enthaltenden Lösung,
(b) in Kontakt bringen der Lösung, welche den Bindungsmediator und die Nukleinsäuren umfasst, mit einer Oberfläche unter chaotropen und/oder Hoch-Salz-Bedingungen
(c) Bindung bzw. Adsorption der Nukleinsäuren an eine Oberfläche,
(d) Waschen der Oberfläche durch einen Waschpuffer,
(e) Wiedergewinnung der Nukleinsäuren, welche an die Oberfläche adsorbiert sind, durch Elution,
**dadurch gekennzeichnet, dass** der Bindungsmediator ausgewählt ist aus der Gruppe umfassend Diethylenglycolmonoethylether, Diethylenglycolmonoethyletheracetat, Furfurylalkohol, Poly(1-vinylpyrrolidon-co-2-dimethylaminoethylmethacrylat), Poly(2-ethyl-2-oxazolin), Poly(4-ammonium-styrolsulfonsäure), Tetraethylenglycoldimethylether, Tetraethylenglycol, Tetrahydrofurfuryl-polyethylenglycol 200 und Triethylenglycolmonoethylether.

2. Verfahren gemäß Anspruch 1, **dadurch gekennzeichnet, dass** die Oberfläche, an welche die Nukleinsäuren adsorbiert werden, auf Materialien basieren, die aus der folgenden Gruppe ausgewählt sind: Silika-Materialien, carboxylierte Oberflächen, Zeolithe und Titandioxid.

3. Verfahren nach einem der Ansprüche 1 oder 2, **dadurch gekennzeichnet, dass** chaotrope Bedingungen durch den Zusatz von chaotropen Salzen, ausgewählt aus der Gruppe umfassend Kaliumiodid, Guanidiniumhydrochlorid, Guanidiniumthiocyanat oder Natriumchlorid zu der Nukleinsäure enthaltenen Lösung erreicht werden.

4. Verfahren gemäß einem der vorhergehenden Ansprüche, **dadurch gekennzeichnet, dass** die Nukleinsäure genomische DNA ist.

5. Verfahren gemäß einem der vorhergehenden Ansprüche, **dadurch gekennzeichnet, dass** die Nukleinsäure Gesamt-RNA ist.

6. Verfahren gemäß einem der vorhergehenden Ansprüche, **dadurch gekennzeichnet, dass** die Nukleinsäuren kurze doppelsträngige DNA Fragmente sind.

7. Verfahren gemäß einem der vorhergehenden Ansprüche, **dadurch gekennzeichnet, dass** die Nukleinsäure-enthaltende Lösung durch einen Lysevorgang aus einem Nukleinsäure enthaltenden Material gewonnen wird.

8. Verfahren gemäß einem der Ansprüche 1 bis 7, **dadurch gekennzeichnet, dass** die Nukleinsäure-enthaltende Lösung aus einer biochemischen Nukleinsäuremodifizierungsreaktion gewonnen wird.

9. Verfahren gemäß einem der Ansprüche 1 bis 8, **dadurch gekennzeichnet, dass** das Nukleinsäure enthaltende Material ausgewählt ist aus der Gruppe umfassend Blut, Gewebe, Abstrichpräparate, Bakterien, Zellsuspensionen und adherierende Zellen, PCR-Reaktionen und *in vitro*-Nukleinsäuremodifikationsreaktionen.

10. Reagenzien-Kit zur Extraktion von Nukleinsäuren aus einer Lösung umfassend
eine Lösung 1 umfassend den Bindungsmediatior ausgewählt aus der Gruppe umfassend Diethylenglycolmonoethylether, Diethylenglycolmonoethyletheracetat, Furfurylalkohol, Poly(1-vinylpyrrolidon-co-2-dimethylaminoethylmethacrylat), Poly(2-ethyl-2-oxazolin), Poly(4-ammonium-styrolsulfonsäure),Tetraethylenglycol-dimethylether, Tetraethylenglycol, Tetrahydrofurfuryl-polyethylenglycol 200 und Triethylenglycolmonoethylether.

11. Reagenzien-Kit zur Extraktion von Nukleinsäuren aus einer Lösung gemäß Anspruch 10 zusätzlich umfassend
eine Lösung 2 umfassend Waschpuffer, und
eine Lösung 3 umfassend ein Elutionsmittel.

12. Reagenzien-Kit zur Extraktion von Nukleinsäuren aus einer Lösung gemäß Anspruch 10 oder 11, umfassend eine weitere Lösung 4 umfassend einen Lysepuffer und eine Protease.

13. Reagenzien-Kit zur Extraktion von Nukleinsäuren aus einer Lösung gemäß einem der Ansprüche 10 bis 12, **dadurch gekennzeichnet, dass** die mindestens eine vorhandene Lyse-Lösung ein chaotropes Salz enthält.

14. Reagenzien-Kit zur Extraktion von Nukleinsäuren aus einer Lösung gemäß Anspruch 13, **dadurch gekennzeichnet, dass** das chaotrope Salz ausgewählt ist aus einer Gruppe umfassend Kaliumiodid, Guanidiniumhydrochlorid, Guanidiniumthiocyanat und Natriumchlorid.

15. Verwendung eines Reagenzien-Kits gemäß einem der Ansprüche 10 bis 14 für die Extraktion von Nukleinsäuren aus biologischen Materialien ausgewählt aus der Gruppe umfassend Blut, Gewebe, Abstrichpräparate, Bakterien, Zellsuspensionen und adherierende Zellen.

16. Verwendung eines Reagenzien-Kits gemäß einem der Ansprüche 10 bis 14 für die Aufreinigung von Nukleinsäuren aus biochemischen Reaktionen, PCR-Reaktionen oder *in vitro*-Nukleinsäuremodifikationensreaktionen.
